# EUROPÄISCHE PATENTSCHRIFT

(11) **EP 2 335 662 B1**
(45) Veröffentlichungstag und Bekanntmachung des Hinweises auf die Patenterteilung: **25.03.2020**
(21) Anmeldenummer: 09015760.3
(22) Anmeldetag: 19.12.2009
(51) Int. Cl.: A61F 13/15, A61F 13/56, B32B 25/10, B32B 25/14, B32B 27/30, A61F 13/49, B32B 7/02, B32B 7/04, B32B 27/12, B32B 5/26

(54) **Elastisches Verbundmaterial**
Elastic compound material
Matériau composite élastique

(43) Veröffentlichungstag der Anmeldung: 22.06.2011
(73) Patentinhaber: Mondi AG, 1030 Wien (AT)
(72) Erfinder: Sollmann, Henner, 48599 Gronau (DE)
(74) Vertreter: Lorenz, Bernd Ingo Thaddeus

(56) Entgegenhaltungen:
- EP-A1- 1 844 928
- EP-A1- 1 900 512
- WO-A1-03/047488
- US-A1- 2004 044 324

## Beschreibung

Die Erfindung betrifft ein elastisches Verbundmaterial mit einer Folie, die an ihren beiden Seiten von ein- oder mehrschichtigen Vliesstoffauflagen abgedeckt ist. Das elastische Verbundmaterial ist insbesondere für stark belastete Bereiche von Hygieneartikeln, wie beispielsweise elastische Verschlussstreifen von Einwegwindeln, vorgesehen.

Styrol-Block-Copolymere zeichnen sich durch gute elastische Eigenschaften aus und sind deshalb für die Herstellung hoch belasteter elastischer Verbundmaterialien geeignet.

Aufgrund der sehr hohen Elastizität weisen Styrol-Block-Copolymere auch eine hohe Klebrigkeit auf und können deshalb nur unter Aufwand als Monofolien verarbeitet werden. Neben einer schwer zu kontrollierenden Dehnung während der Verarbeitung einer solchen Folie kann diese auch nicht ohne Weiteres auf eine Rolle aufgerollt werden, da dann die erhöhte Gefahr eines Verblockens besteht.

Aus der DE 298 25 018 U1 ist ein dehnbarer elastischer Streifen bekannt, der eine coextrudierte, elastische Folie aufweist. Die elastische Folie umfasst eine elastische Schicht sowie zumindest eine weitere, nicht elastische Schicht. Bei dem dehnbar elastischen Streifen ist die Coextrusionsfolie an zumindest einer Seite durch eine Vliesauflage abgedeckt, wobei gezielt dehnbare und nicht dehnbare Bereiche erzeugt werden. Dabei kann vorgesehen sein, dass in den dehnbaren Bereichen die Vliesstoffauflage an voneinander beabstandeten Befestigungsbereichen mit der Coextrusionsfolie verbunden ist, wobei sich zwischen den Befestigungsbereichen aufgeworfene bogenförmige Bereiche bilden. Durch den Wechsel von festgelegten Befestigungsbereichen und bogenförmigen Bereichen bildet sich eine deutlich erkenn- und fühlbare Wellenform aus, wodurch die Haptik und der textile Charakter des Materials beeinträchtigt werden.

Vor diesem Hintergrund liegt der Erfindung die Aufgabe zugrunde, ein elastisches Verbundmaterial anzugeben, welches an einer Seite eine gleichmäßige, besonders weiche Oberfläche aufweist.

Gegenstand der Erfindung und Lösung der Aufgabe ist ein elastisches Verbundmaterial mit einer Folie, die an ihren beiden Seiten von ein- oder mehrschichtigen Vliesstoffauflagen abgedeckt ist, wobei die Folie als Coextrusionsfolie ausgebildet ist, deren erste Oberfläche von einer ersten, elastischen und klebrigen Folienschicht aus Styrol-Block-Copolymer gebildet ist und deren gegenüberliegende zweite Oberfläche von einer zweiten Folienschicht gebildet ist, die eine geringere Elastizität als die erste Folienschicht aufweist, wobei zwischen der ersten elastischen Folienschicht und einer ersten Vliesstoffauflage eine erste vollflächige Klebstoffschicht angeordnet ist, wobei zwischen der zweiten Folienschicht und einer zweiten Vliesstoffauflage eine zweite zumindest im Wesentlichen vollflächige Klebstoffschicht angeordnet ist, wobei die Verbundhaftung zwischen der zweiten Folienschicht und der zweiten Klebstoffschicht kleiner ist als die Verbundhaftung zwischen der ersten Folienschicht und der ersten Klebstoffschicht und wobei das Verbundmaterial Bereiche aufweist, in denen die zweite Klebstoffschicht von der zweiten Folienschicht und/oder die zweite Folienschicht von der ersten Folienschicht abgelöst ist.

Das elastische Verbundmaterial ist durch eine erstmalige Dehnung aktiviert, wobei erfindungsgemäß bereichsweise eine Ablösung der zweiten Klebstoffschicht von der zweiten Folienschicht und/oder der zweiten Folienschicht von der ersten Folienschicht erfolgt. Vor der Aktivierung sind sowohl die erste Vliesstoffauflage mit der ersten Folienschicht als auch die zweite Vliesstoffauflage mit der zweiten Folienschicht durch die jeweils zugeordnete Klebstoffschicht vollflächig verbunden. Die Verbundhaftung ist dabei so eingestellt, dass bei der Aktivierung durch ein erstmaliges Dehnen sich die zweite Folienschicht bereichsweise von der zweiten Klebstoffschicht und/oder der ersten Folienschicht ablöst. Die Verbundhaftung zwischen der zweiten Folienschicht und der zweiten Klebstoffschicht ist üblicherweise auch geringer als die kohäsiven Bindungskräfte innerhalb der zweiten Klebstoffschicht sowie auch geringer als die Verbundhaftung zwischen den Klebstoffschichten einerseits und der jeweils zugeordneten Vliesstoffauflage andererseits. Bei der Dehnung erfolgt dann bereichsweise ein Adhäsionsbruch zwischen der zweiten Folienschicht und der ersten Folienschicht und/oder der zweiten Folienschicht und der zweiten Klebstoffschicht. Durch die bereichsweise Ablösung innerhalb des Schichtverbundes wird eine Mikrostruktur ohne ein festes Muster erzeugt, wodurch sich unter der zweiten Vliesstoffauflage über die gesamte Fläche des elastischen Verbundmaterials verteilt kleine Aufwerfungen ergeben, welche die haptischen Eigenschaften der zweiten Vliesstoffauflage verbessern. So kann im Vergleich zu einer Ausgestaltung, bei der keine bereichsweise Schichtrennung erfolgt, bei einem gleichen Flächengewicht der zweiten Vliesstoffauflage eine weichere, voluminösere Oberfläche erzeugt werden.

Die Vliesstoffauflage kann aus einer Schicht oder aus mehreren Schichten eines kardierten Vlieses, eines Spinnvlieses oder eines Meltblownvlieses gebildet sein, wobei insbesondere auch mehrschichtige Kombinationen unterschiedlicher Vliesmaterialien in Betracht kommen. Aufgrund der geringen Kosten sind insbesondere für Wegwerfartikel Polyolefine wie Polyethylen oder Polypropylen, Polyolefin-Copolymere oder Polyolefin-Mischungen als Grundsubstanz der Fasern bevorzugt.

Die vorliegende Erfindung beruht auf der Erkenntnis, dass durch die Einstellung der Verbundhaftung an den Grenzflächen der zweiten Folienschicht die Haptik in Bezug auf die zweite Vliesstoffauflage verbessert werden kann. Die erfindungsgemäße Schichttrennung kann durch eine geeignete Abstimmung der Polaritäten der einzelnen Schichten erfolgen, wobei große Polaritätsunterschiede allgemein zu einer Reduzierung der Verbundhaftung führen. Das Styrol-Block-Copolymer als Material für die erste Folienschicht weist eine mittlere Polarität auf. Zusätzlich wird gemäß einer bevorzugten Ausgestaltung der Erfindung als Material für die Klebstoffschichten ein Heißschmelzklebstoff auf der Basis von Styrol-Block-Copolymer, insbesondere Styrol-Isopren-Styrol-Block-Copolymer (SIS) eingesetzt, wobei grundsätzlich beide Klebstoffschichten aus dem gleichen Klebstoff gebildet sein können. Darüber hinaus können auch Klebstoffe auf der Basis von Polyurethan (PU) Verwendung finden. Um einen bereichsweisen Adhäsionsbruch an einer der Grenzflächen der zweiten Folienschicht zu erreichen, ist zweckmäßigerweise ein Material auszuwählen, welches sich in der Polarität deutlich von den angrenzenden Schichten unterscheidet. Da Styrol-Block-Copolymere eine mittlere Polarität aufweisen, sind also entweder stark polare Polymere sowie gering polare oder unpolare Polymere für die zweite Folienschicht geeignet.

Als Polymere mit einer starken Polarität sind beispielsweise Polyamide (PA) mit ihrer stark polaren Amidgruppe, Polymethylmethacrylat (PMMA), Polyoxymethylen (POM) sowie Esther-Thermoplaste, wie Polycarbonat (PC), Polyethylenterephtalat (PET) und Polybutylenterephthalat (PBT) geeignet. Des Weiteren können auch zumindest zwei der genannten Polymere und/oder verschiedene Typen der genannten Polymere als Mischung vorgesehen sein.

Je nach Schichtzusammenstellung kann die gewünschte Trennung auch noch mit einem mäßig polaren Polymer in Kombination mit einer angrenzenden Schicht aus gering polarem oder unpolarem Polymer erreicht werden. Eine je nach Materialkombination noch ausreichend hohe Polarität weist beispielsweise Polystyrol (PS) auf, dessen mäßige Polarität auf das π-Elektronensystem der aromatischen Bestandteile zurückzuführen ist.

Als nicht oder nur gering polare Materialien sind insbesondere Polyolefine wie Polyethylen (PE) und Polypropylen (PP) geeignet.

Zweckmäßigerweise werden die Polymere so ausgewählt, dass der Polaritätsunterschied zwischen der ersten Folienschicht und der ersten Klebstoffschicht geringer ist als einerseits der Polaritätsunterschied zwischen der ersten Folienschicht und der zweiten Folienschicht und andererseits der Polaritätsunterschied zwischen der zweiten Folienschicht und der zweiten Klebstoffschicht.

Die innere Struktur des Verbundmaterials nach der Aktivierung hängt insbesondere von den Materialeigenschaften der zweiten Folienschicht und der Art des Aktivierungsprozesses ab. Bei der Aktivierung erfolgt üblicherweise eine Dehnung von mindestens 50 %. Bevorzugt erfolgt aber eine Dehnung um zumindest 100 %, wobei auch eine deutlich stärkere Längenzunahme vorgesehen sein kann. Die Aktivierung kann nur in eine Richtung erfolgen, um eine bevorzugte Dehnungsrichtung festzulegen. Insbesondere besteht auch die Möglichkeit, ein Material zu erzeugen, welches senkrecht zur Aktivierungsrichtung bei mäßigen Zugkräften im Wesentlichen steif ist.

Gemäß einer ersten weiteren Ausgestaltung bleibt die Folienschicht auch nach der Aktivierung vollständig in sich geschlossen. Im Rahmen einer solchen Ausgestaltung wird die zweite Folienschicht während der Aktivierung plastisch verformt ohne zu zerreißen. Eine abschnittsweise Schichttrennung kann dabei sowohl gegenüber der ersten Folienschicht als auch der zweiten Folienschicht erfolgen.

Gemäß einer alternativen Ausgestaltung weist die zweite Folienschicht aufgebrochene Stellen auf. Diese bilden sich, wenn die zweite Folienschicht während der Aktivierung den Zugkräften nicht standhalten kann und einreißt. Da die zweite Folienschicht sowohl durch die über die zweite Klebstoffschicht angrenzende zweite Vliesstoffauflage als auch die erste Folienschicht in einem gewissen Maße gestützt und geführt wird, treten in der Regel flächig verteilte Mikrorisse auf, ohne dass die zweite Folienschicht vollständig zerreißt.

Die vorzugsweise lediglich zweischichtige Coextrusionsfolie weist üblicherweise eine Gesamtdicke zwischen 12 µm und 150 µm auf. Die Dicke der ersten, elastischen Folienschicht liegt üblicherweise zwischen 10 µm und 100 µm, vorzugsweise zwischen 25 µm und 70 µm. Die zweite Folienschicht weist üblicherweise eine Dicke zwischen 2 µm und 50 µm, vorzugsweise zwischen 5 µm und 20 µm auf. In diesem Zusammenhang ist zu berücksichtigen, dass die elastischen Eigenschaften des Verbundmaterials im Wesentlichen durch die erste elastische Folienschicht bestimmt und Einschränkungen der Elastizität durch die zweite Folienschicht vermieden werden sollen. Bei einer ausreichenden Festigkeit der zweiten Folienschicht kann diese auch die Coextrusionsfolie als Vorprodukt während der Verarbeitung stabilisieren, wobei während der Handhabung der Coextrusionsfolie unter Zug eine starke Dehnung der Coextrusionsfolie vermieden werden kann. Insbesondere kann die Coextrusionsfolie als Vorprodukt auch aufgerollt und gelagert werden, da die zweite, nicht klebrige Folienschicht als Trennung zwischen den aufeinander liegenden Lagen der ersten Folienschicht ein Verblocken vermeidet.

Die Verbundhaftung kann nach DIN 53357: 1982 bestimmt werden.

Aus dem elastischen Verbundmaterial können Streifen abgeschnitten werden, die im besonderen Maße als elastische Verschlussstreifen von Windeln eingesetzt werden können.

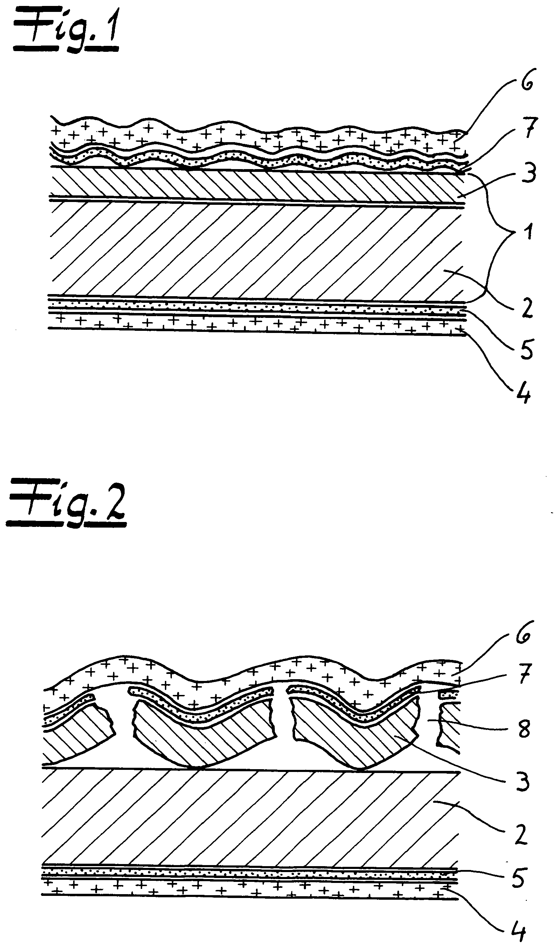

Die Erfindung wird im Folgenden anhand einer lediglich ein Ausführungsbeispiel darstellenden Zeichnung erläutert. Die Fig. 1 und 2 zeigen unterschiedliche Ausgestaltungen des erfindungsgemäßen Verbundmaterials.

Gemäß der Fig. 1 weist das elastische Verbundmaterial eine zweischichtige Coextrusionsfolie 1 mit einer ersten, klebrigen Folienschicht 2 aus Styrol-Block-Copolymer und eine zweite Folienschicht 3 auf, die eine geringere Elastizität als die erste Folienschicht 2 aufweist. Eine erste Vliesstoffauflage 4 ist durch eine erste Klebstoffschicht 5 vollflächig mit der ersten Folienschicht 2 verbunden.

Zwischen der zweiten Folienschicht 3 und einer zweiten Vliesstoffauflage 6 ist eine zweite Klebstoffschicht 7 angeordnet, die bereichsweise von der zweiten Folienschicht 3 abgelöst ist, aber noch an dieser anhaftet. Diese bereichsweise Ablösung wird durch ein erstmaliges Dehnen des Verbundmaterials, beispielsweise unter einer Erhöhung der Länge um 100 %, erreicht. Bei der im Wesentlichen vollständigen Rückstellung des Verbundmaterials aufgrund der elastischen Eigenschaften der ersten Folienschicht 2 zieht sich das Material wieder zusammen, wobei sich aufgrund der geringen Verbundhaftung an der gemeinsamen Grenzfläche die zweite Folienschicht 3 von der zweiten Klebstoffschicht 7 bereichsweise durch einen Adhäsionsbruch trennt. Durch die Aufwerfungen der zweiten Vliesstoffauflage 6 und der zweiten Klebstoffschicht 7 wird die zweite Vliesstoffauflage 6 insgesamt voluminöser, ohne dass für einen Benutzer eine makroskopische Struktur erkennbar ist. Vielmehr wird bereits bei einem geringen Flächengewicht der zweiten Vliesstoffauflage 6 eine angenehme Haptik erreicht.

Fig. 2 zeigt eine Ausgestaltung mit einem grundsätzlich vergleichbaren Schichtaufbau, wobei das Material der zweiten Folienschicht so ausgewählt ist und die Aktivierung derart ausgeführt wird, dass die zweite Folienschicht 3 bereichsweise einreißt, so dass nach der Aktivierung aufgebrochene Stellen 8 in der zweiten Folienschicht 3 verbleiben. Des Weiteren ist die Polarität der zweiten Folienschicht derart auf das Material der ersten Folienschicht abgestimmt, dass an der Grenzfläche zwischen den Folienschichten 2, 3 eine bereichsweise Ablösung erfolgt. Wie zuvor in Bezug auf Fig. 1 beschrieben, bewirkt die teilweise Ablösung eine Mikrostrukturierung unterhalb der zweiten Vliesstoffauflage 6, wodurch diese weicher und voluminöser wirkt.

## Patentansprüche

1. Elastisches Verbundmaterial mit einer Folie, die an ihren beiden Seiten von ein- oder mehrschichtigen Vliesstoffauflagen abgedeckt ist,
wobei die Folie als Coextrusionsfolie (1) ausgebildet ist, deren erste Oberfläche von einer ersten, elastischen Folienschicht (2) aus Styrol-Block-Copolymer gebildet ist und deren gegenüberliegende zweite Oberfläche von einer zweiten Folienschicht (3) gebildet ist, die eine geringere Elastizität als die erste Folienschicht (2) aufweist,
wobei zwischen der ersten, elastischen Folienschicht (2) und einer ersten Vliesstoffauflage (4) eine erste Klebstoffschicht (5) angeordnet ist,
wobei zwischen der zweiten Folienschicht (3) und einer zweiten Vliesstoffauflage (6) eine zweite Klebstoffschicht (7) angeordnet ist,
wobei die Verbundhaftung zwischen der zweiten Folienschicht (3) und der zweiten Klebstoffschicht (7) kleiner ist als die Verbundhaftung zwischen der ersten Folienschicht (2) und der ersten Klebstoffschicht (5) und
wobei das Verbundmaterial Bereiche aufweist, in denen die zweite Klebstoffschicht (7) von der zweiten Folienschicht (3) und/oder in denen die zweite Folienschicht (3) von der ersten Folienschicht (2) abgelöst ist.

2. Verbundmaterial nach Anspruch 1, wobei die erste Klebstoffschicht (5) und die zweite Klebstoffschicht (7) aus dem gleichen polymeren Material bestehen.

3. Verbundmaterial nach Anspruch 1oder 2, wobei die erste Klebstoffschicht (5) und die zweite Klebstoffschicht (7) aus einem Heißschmelzklebstoff auf der Basis von Styrol-Block-Copolymer bestehen.

4. Verbundmaterial nach einem der Ansprüche 1 bis 3, wobei die zweite Folienschicht (3) als polymere Grundsubstanz ein Polymer ausgewählt aus der Gruppe Polyamid (PA), Polymethylmethacrylat (PMMA), Polyoxymethylen (POM), Polycarbonat (PC), Polyethylenterephtalat (PET), Polybutylenterephthalat (PBT) oder eine Mischung zumindest zwei dieser Polymere aufweist.

5. Verbundmaterial nach einem der Ansprüche 1 bis 3, wobei die zweite Folienschicht (3) als polymere Grundsubstanz Polyolefin, insbesondere Polyethylen (PE) und/oder Polypropylen (PP) aufweist.

6. Verbundmaterial nach einem der Ansprüche 1 bis 5, wobei der Polaritätsunterschied zwischen der ersten Folienschicht (2) und der ersten Klebstoffschicht (5) geringer ist als einerseits der Polaritätsunterschied zwischen der ersten Folienschicht (2) und der zweiten Folienschicht (3) und andererseits der Polaritätsunterschied zwischen der zweiten Folienschicht (3) und der zweiten Klebstoffschicht (7).

7. Verbundmaterial nach einem der Ansprüche 1 bis 6, wobei die erste und die zweite Vliesstoffauflage (4, 6) gleich ausgebildet sind.

8. Verbundmaterial nach einem der Ansprüche 1 bis 7, wobei die erste, elastische Folienschicht (2) eine Dicke zwischen 10 µm und 100 µm aufweist.

9. Verbundmaterial nach einem der Ansprüche 1 bis 8, wobei die zweite Folienschicht (3) eine Dicke zwischen 2 µm und 50 µm aufweist.

10. Verbundmaterial nach einem der Ansprüche 1 bis 9, wobei die Coextrusionsfolie (1) zweischichtig ist.

11. Verbundmaterial nach einem der Ansprüche 1 bis 10, wobei die Verarbeitungstemperatur der Polymere der ersten Folienschicht (2), der zweiten Folienschicht (3) und der Klebstoffschichten zwischen 180 °C und 260 °C liegen.

12. Verbundmaterial nach einem der Ansprüche 1 bis 11, wobei die zweite Folienschicht (3) vollflächig in sich geschlossen ist.

13. Verbundmaterial nach einem der Ansprüche 1 bis 11, wobei die zweite Folienschicht (3) aufgebrochene Stellen (8) aufweist.

## Claims

1. An elastic composite material comprising a film which is covered on both its sides by single- or multi-layer nonwoven overlays,
wherein the film is configured as co-extruded film (1), the first surface of which is formed by a first elastic film layer (2) of styrene block copolymer and the opposite second surface of which is formed by a second film layer (3) which has a lower elasticity than the first film layer (2),
wherein a first adhesive layer (5) is arranged between the first elastic film layer (2) and a first nonwoven overlay (4),
wherein a second adhesive layer (7) is arranged between the second film layer (3) and a second nonwoven overlay (6),
wherein the composite adhesion between the second film layer (3) and the second adhesive layer (7) is weaker than the composite adhesion between the first film layer (2) and the first adhesive layer (5) and
wherein the composite material has regions in which the second adhesive layer (7) is separated from the second film layer (3) and/or in which the second film layer (3) is separated from the first film layer (2).

2. The composite material according to claim 1, wherein the first adhesive layer (5) and the second adhesive layer (7) consist of the same polymer material.

3. The composite material according to claim 1 or 2, wherein the first adhesive layer (5) and the second adhesive layer (7) consist of a hot-melt adhesive based on a styrene block copolymer.

4. The composite material according to one of claims 1 to 3, wherein the second film layer (3) comprises as polymer basic substance a polymer selected from the group of polyamide (PA), polymethyl methacrylate (PMMA), polyoxymethylene (POM), polycarbonate (PC), polyethylene terephthalate (PET), polybutylene terephthalate (PBT) or a mixture of at least two of these polymers.

5. The composite material according to one of claims 1 to 3, wherein the second film layer (3) comprises as polymer basic substance polyolefin, in particular polyethylene (PE) and/or polypropylene (PP).

6. The composite material according to one of claims 1 to 5, wherein the polarity difference between the first film layer (2) and the first adhesive layer (5) is less than on the one hand the polarity difference between the first film layer (2) and the second film layer (3) and on the other hand the polarity difference between the second film layer (3) and the second adhesive layer (7).

7. The composite material according to one of claims 1 to 6, wherein the first and the second nonwoven overlay (4, 6) are configured to be the same.

8. The composite material according to one of claims 1 to 7, wherein the first elastic film layer (2) has a thickness between 10 µm and 100 µm.

9. The composite material according to one of claims 1 to 8, wherein the second film layer (3) has a thickness between 2 µm and 50 µm.

10. The composite material according to one of claims 1 to 9, wherein the co-extruded film (1) is two-layered.

11. The composite material according to one of claims 1 to 10, wherein the processing temperature of the polymers of the first film layer (2), the second film layer (3) and the adhesive layers are between 180°C and 260°C.

12. The composite material according to one of claims 1 to 11, wherein the second film layer (3) is closed in itself over the entire area.

13. The composite material according to one of claims 1 to 11, wherein the second film material (3) has broken locations (8).

## Revendications

1. Matière composite élastique pourvue d'un film, qui sur ses deux faces est recouvert de supports en non-tissé monocouche ou multicouches,
le film étant conçu sous la forme d'un film coextrudé (1) dont la première surface est formée d'une première couche de film (2) élastique en un copolymère styrénique et dont la deuxième surface opposée est formée d'une deuxième couche de film (3), qui fait preuve d'une élasticité moindre à celle de la première couche de film (2),
entre la première couche de film (2) élastique et un premier support en non-tissé (4) étant placée une première couche d'agent adhésif (5),
entre la deuxième couche de film (3) et un deuxième support en non-tissé (6) étant placée une deuxième couche d'agent adhésif (7),
la liaison composite entre la deuxième couche de film (3) et la deuxième couche d'agent adhésif (7) étant moindre que la liaison composite entre la première couche de film (2) et la première couche d'agent adhésif (5) et
la matière composite comportant des zones dans lesquelles la deuxième couche d'agent adhésif (7) est désolidarisée de la deuxième couche de film (3) et/ou dans lesquelles la deuxième couche de film (3) est désolidarisée de la première couche de film (2).

2. Matière composite selon la revendication 1, la première couche d'agent adhésif (5) et la deuxième couche d'agent adhésif (7) étant constituées de la même matière polymère.

3. Matière composite selon la revendication 1 ou 2, la première couche d'agent adhésif (5) et la deuxième couche d'agent adhésif (7) étant constituées d'une matière adhésive thermofusible sur la base de copolymère styrénique.

4. Matière composite selon l'une quelconque des revendications 1 à 3, la deuxième couche de film (3) comportant en tant que substance de base polymère un polymère sélectionné dans le groupe comprenant le polyamide (PA), le polyméthacrylate de méthyle (PMMA), le polyoxyméthylène (POM), le polycarbonate (PC), le polyéthylène téréphtalate (PET), le polybutylène téréphtalate (PBT) ou un mélange d'au moins deux desdits polymères.

5. Matière composite selon l'une quelconque des revendications 1 à 3, la deuxième couche de film (3) comportant en tant que substance de base polymère une polyoléfine, notamment un polyéthylène (PE) et/ou un polypropylène (PP).

6. Matière composite selon l'une quelconque des revendications 1 à 5, la différence de polarité entre la première couche de film (2) et la première couche d'agent adhésif (5) étant inférieure à d'une part la différence de polarité entre la première couche de film (2) et la deuxième couche de film (3) et d'autre part la différence de polarité entre la deuxième couche de film (3) et la deuxième couche d'agent adhésif (7).

7. Matière composite selon l'une quelconque des revendications 1 à 6, les premier et deuxième supports en non-tissé (4, 6) étant conçus de manière identique.

8. Matière composite selon l'une quelconque des revendications 1 à 7, la première couche de film (2) élastique présentant une épaisseur comprise entre 10 µm et 100 µm.

9. Matière composite selon l'une quelconque des revendications 1 à 8, la deuxième couche de film (3) présentant une épaisseur comprise entre 2 µm et 50 µm.

10. Matière composite selon l'une quelconque des revendications 1 à 9, le film coextrudé (1) étant bicouches.

11. Matière composite selon l'une quelconque des revendications 1 à 10, la température de mise en œuvre des polymères de la première couche de film (2), de la deuxième couche de film (3) et des couches d'agent adhésif se situant entre 180 °C et 260 °C.

12. Matière composite selon l'une quelconque des revendications 1 à 11, la deuxième couche de film (3) étant fermée en soi à pleine surface.

13. Matière composite selon l'une quelconque des revendications 1 à 11, la deuxième couche de film (3) comportant des endroits (8) rompus.
